# EUROPEAN PATENT SPECIFICATION

(11) **EP 4 384 037 B1**
(45) Date of publication and mention of the grant of the patent: **10.06.2026**
(21) Application number: 22754957.3
(22) Date of filing: 05.08.2022
(51) Int. Cl.: A41D 13/06, A41D 13/015, A61F 5/01

(54) **KNEE BRACE**
KNIESCHIENE
ATTELLE DE GENOU

(30) Priority: 09.08.2021 IT 202100021542
(43) Date of publication of application: 19.06.2024
(73) Proprietor: Alpinestars Research S.p.A., 31010 Maser (TV) Frazione: Coste (IT)
(72) Inventor: MAZZAROLO, Giovanni, 31010 Coste di Maser (Treviso) (IT); BORSATO, Stefano, 31044 Biadene di Montebelluna (Treviso) (IT); PIOVESAN, Paolo, 31010 Maser (Treviso) (IT)
(74) Representative: Dragotti & Associati S.P.A.
(86) International application number: PCT/IB2022/057299
(87) International publication number: WO 2023/017382

(56) References cited:
- WO-A1-2008/061300
- WO-A1-2016/081797
- US-A1- 2008 195 013

## Description

The present invention relates to a knee brace. In particular, the present invention relates to a knee brace designed to protect the knee joint of a user during sport activities, like for example motorcycling, cycling, skiing, etc.

Nowadays the use of knee braces is largely diffused during sport activities.

The knee braces have been studied to protect users from injuries coming from impacts and unnatural movements of the knee, like unnatural flexion, torsion or extension movements.

Generally, the known knee braces comprise an upper frame member, designed to be fastened to the upper part of the user's leg, and a lower frame member, designed to be fastened to the lower part of the user's leg.

The upper frame member and the lower frame member are connected to each other by means of a single or double hinge which enables the frame members to follow the flexion movement of the leg, preventing at the same time knee injuries coming from hyper-rotation, hyper-flexion or hyper-extension of the knee joint.

In motorcycling application, in view of the high forces involved, the frame members are also made of rigid material, so as to offer to the knee an improved protection against direct impacts.

Moreover, to offer an additional protection to the patella of the user against impacts, the knee braces can also be provided with a semi-rigid or rigid protecting cup, designed to be superimposed in use over the user's patella.

Such a protecting cup is connected to the rigid frame members by means of a slidable mechanism which has the function to maintain the protecting cup in the correct position during the knee brace's movements. The slidable mechanism is usually coupled to the articulation hinge of the knee brace.

Even if the above mentioned knee braces are greatly appreciated, they have some drawbacks.

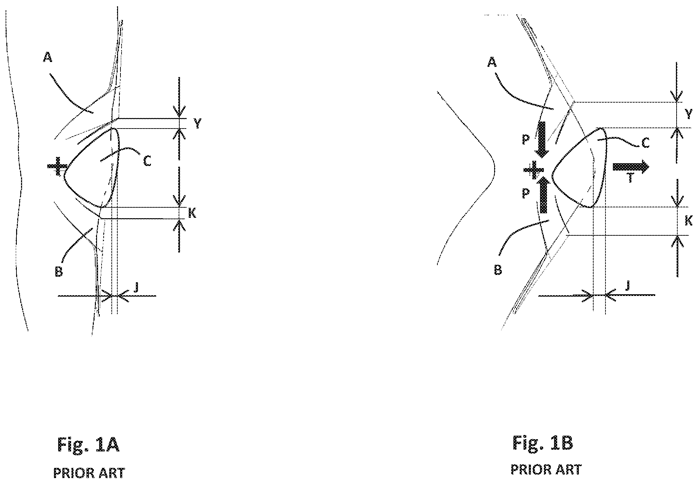

As schematically shown in figures 1A-1B, which are enclosed to the present description to better illustrate the technical problem faced and solved by the present invention, the protection of the patella of the user is critical during the movements of the knee brace.

As a matter of fact, during the passage from the configuration of figure 1A, schematically showing a knee brace in a straight configuration, to the configuration of figure 1B, schematically showing a knee brace in a bent configuration, both the gap Y, between the upper frame member A and the protecting cup C, and the gap K, between the lower frame member B and the protecting cup C, increase, leaving a larger area of the patella of the user not protected.

This is due to the fact that the movements of the upper and lower frame members A, B are independent from the movements of the protecting cup C and that, when the knee brace is bending, such frame members A, B move away from the protecting cup C.

Moreover, the relative movement of the upper and lower parts of the leg is not a pure rotation, but a combination of different linear and rotational movements which result in a roto-translational movement.

Even if the use of hinges having at least two centers of rotation, the so-called polycentric hinge, is known, these hinges are still not able to perfectly adapt to the roto-translational movements of the knee joint.

Referring again to figures 1A-1B, when the frames A, B are fastened to the user's leg and the leg is flexed, the movement of the leg would cause a reduction of the distance between the facing ends of the upper frame A and lower frame B.

However, these ends are connected to the articulation hinge and thus are mutually blocked, consequently a reduction of the distance between these facing ends (schematically shown in figure 1B by the arrows P) results in an outward movement of the articulation hinge and therefore in an outward movement of the protecting cup C. Such a movement is schematically shown in figure 1B by the arrow T.

The outward movement of the articulation hinge contributes in having bigger gaps Y, K, between the protecting cup C and the knee brace frames A, B, and in a bigger gap J, between the protecting cup C and the patella of the user.

Obviously, the presence of these gaps Y, K, J affects the protection offered by the knee brace to the knee of the user against frontal and side impacts.

Moreover, the presence of these gaps Y, K, J also produces a discontinuity between the upper and lower frame members A, B and the protecting cup C. Consequently, the edges of the protecting cup C are visible under the pants, which are generally worn by the user over the knee brace, and most importantly they can act like an "edge" with the risk to become a penetrating element in case an impact occurs when the knee brace is in the bent configuration.

US9693887 discloses a knee brace which comprises a protecting cup designed to provide more continuous coverage of the user's patella.

In detail, the protecting cup disclosed in US9693887 comprises: an upper cup element, coupled to the upper frame member; a lower cup element, coupled to the lower frame member, and an intermediate cup element, operatively coupled to the upper cup element and lower cup element.

The protecting cup is transitional between a first configuration and a second configuration, wherein a degree of overlap between the upper cup element, the lower cup element and the intermediate cup element decreases as the protecting cup transitions from the first configuration to the second configuration.

Even if such a protecting cup provides more continuous coverage of the user's patella when the knee brace moves from a straight configuration to a bent configuration, the associated knee brace still has further drawbacks.

A first drawback is that the upper cup element and the lower cup element can laterally move or block during sliding, thus preventing the correct functioning of the knee brace.

A further drawback is that the knee brace does not allow an effective breathability of the user's leg, since the cup elements have a closed structure.

Another drawback is that the intermediate member has a reduced deformability and doesn't allow an efficient absorption of the strikes and blows acting against the knee brace.

Furthermore, another drawback is that the upper cup element and the lower cup element are made of a rigid material and could not be deformed at all during sliding, thus preventing the adaptation of the system over the user's leg.

An improved knee brace is disclosed in WO2020/038987 in the name of the same applicant.

The protecting cup of this knee brace has an intermediate member having a double wall construction. Such an intermediate member is provided with channels through which flexible sliding fingers, that are mounted on the upper frame member and lower frame member, can slide.

Thanks to its double wall construction, the protecting cup of WO2020/038987 is able to absorb high impact forces.

At the same time, the sliding fingers are helpful in guiding the mutual movements of the upper and lower frame members during the movements of the knee joint.

Moreover, the sliding fingers, being not cup-shaped, allows an improved ventilation of the leg of the user

However, precisely because the sliding fingers are not cup-shaped, they do not provide a patella impact protection function.

Consequently the area external to the intermediate member of the protecting cup offers a reduced protection, in particular in a bending configuration like the one schematically disclosed in figure 1B.

The object of the present invention is to provide a knee brace which solves at least partly the above mentioned problems and drawbacks.

In particular, an aim of the present invention is to provide a knee brace suitable for offering in all the operative configurations an improved protection to the patella of the user.

Moreover, an aim of the present invention is to provide a knee brace with upper and lower frame members suitable for following the movements of the leg of the user without opposing it.

Furthermore, an aim of the present invention is to provide a knee brace which, even in a bent condition, does not have protruding edges or surfaces.

A further aim of the present invention is to provide a knee brace suitable for offering an improved comfort to the user, in particular improved ventilation of the leg, and for assuring a high stability to the knee joint. WO2008061300 discloses a knee brace wherein a patella protecting cup is provided with a liner made of neoprene or ethylene-vinyl acetate.

These and other objects and aims are achieved by the knee brace according to claim 1.

The advantages and the characteristic features of the invention will appear more clearly from the following description of preferred, but not exclusive, embodiments of the knee brace with reference to the accompanying figures in which:
- figures 1A and 1B schematically shown a prior art knee brace in two different operative configurations;
- figure 2 shows a front view of a first embodiment of the knee brace according to the present invention in a first operative configuration;
- figure 3 is a cross section view taken along the line III-III of figure 2;
- figure 3A is a view similar to figure 3, but relating to a different embodiment;
- figure 4 is an enlarged view of the detail D of figure 3;
- figure 5 shows a first side view of the knee brace of figure 2;
- figure 6 shows an inner view of the knee brace of figure 2;
- figure 7 shows a second side view of the knee brace of figure 2;
- figure 8 shows a perspective view of a component of the knee brace of figure 2;
- figure 9 is a cross section view taken along the line IX-IX of figure 8;
- figure 10 is a perspective view, partially exploded, of a further detail of the knee brace of figure 2;
- figure 10A is a side view of the detail of figure 10;
- figure 10B is a cross section view of a particular of figure 10A taken along the line XB-XB;
- figure 11 shows a first perspective exploded view of the knee brace of figure 2;
- figure 12 shows a second perspective exploded view of the knee brace of figure 2;
- figure 13 shows a front view of the knee brace of figure 2, in a second operative configuration;
- figure 14 shows a side view of the knee brace of figure 13;
- figure 15 is a cross section view taken along the line XV-XV of figure 13;
- figure 16 shows a front view of a second embodiment of the knee brace according to the present invention in a first operative configuration;
- figure 17 is a cross section view taken along the line XVII-XVII of figure 16;
- figure 18 is an enlarged view of the detail E of figure 17;
- figure 19 shows a first side view of the knee brace of figure 16;
- figure 20 shows an inner view of the knee brace of figure 16;
- figure 21 shows a second side view of the knee brace of figure 16;
- figure 22 shows a front view of the knee brace of figure 16, in a second operative configuration;
- figure 23 shows a side view of the knee brace of figure 22;
- figure 24 is a cross section view taken along the line XXIV-XXIV of figure 22;
- figure 25 shows a front view of a third embodiment of the knee brace according to the present invention in a first operative configuration;
- figure 26 is a cross section view taken along the line XXVI-XXVI of figure 25;
- figure 27 is an enlarged view of the detail F of figure 26;
- figure 28 shows a first side view of the knee brace of figure 25;
- figure 29 shows an inner view of the knee brace of figure 25;
- figure 30 shows a second side view of the knee brace of figure 25;
- figure 31 shows a front view of the knee brace of figure 25, in a second operative configuration;
- figure 32 shows a side view of the knee brace of figure 31;
- figure 33 is a cross section view taken along the line XXXIII-XXXIII of figure 31.

In the following description of the knee brace according to the invention, as "upper" there will be indicated the portion of the different components of the knee brace which, during the normal use, is relatively distant from the ground and as "lower" the portion of the different components of the knee brace relatively closer to the ground. Similarly, as "inner" there will be indicated the portion of the different components of the knee brace which, during the normal use, is relatively near to the body of the user and as "outer" the portion of the different components of the knee brace relatively farther.

Finally, as "straight configuration" of the knee brace there will be indicated a configuration wherein the upper frame member and the lower frame member of the knee brace are substantially aligned as shown for example in figure 1A and as "bent configuration" of the knee brace there will be indicated a configuration wherein the upper frame member and the lower frame member of the knee brace are tilted to each other as shown for example in figure 1B.

With reference to the enclosed figures, a knee brace according to the invention is indicated as a whole by the reference 10.

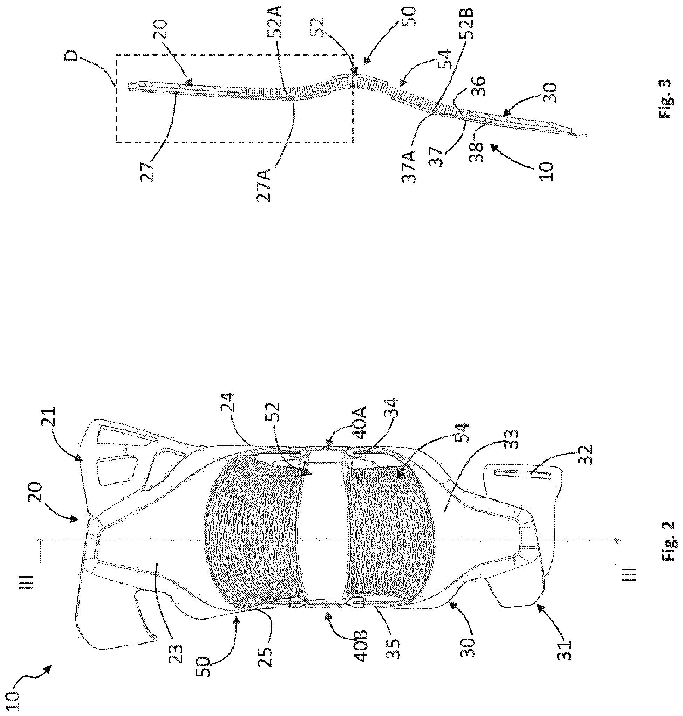

As shown for example in figures 2, 16 and 25, the knee brace 10 comprises an upper frame member 20, designed for being fastened to the upper part of the user's leg, and a lower frame member 30, designed for being fastened to the lower part of the user's leg.

The upper frame member 20 and the lower frame member 30 are pivotally connected to each other by means of at least one articulation hinge 40.

The knee brace 10 also comprises a patella protecting device 50 which is positioned between the upper frame member 20 and the lower frame member 30.

The patella protecting device 50 comprises a protecting cup 52, coupled to the at least one articulation hinge 40 and designed to be positioned over the patella of the user.

According to the invention, the patella protecting device 50 further comprises an elastomeric protecting element 54 which extends between the upper frame member 20 and the lower frame 30 and is operatively coupled to the protecting cup 52.

As shown in the enclosed figures, the elastomeric protecting element 54 can be advantageously coupled to the upper frame member 2 and to the lower frame member 30.

As it will appear clearly from the following description, the operative coupling of the elastomeric protecting element 54 to the protecting cup 52 provides an improved protection to the knee and leg of the user. In particular, the elastomeric protecting element 54 is able to adequately protect a larger area of the knee and leg of the user, even when the knee brace 10 assumes a bent configuration.

Moreover, the combination of the elastomeric protecting element 54 with the protecting cup 52 offers an improved protection against impact forces of high magnitude, since the protection offered by the protecting cup 52 is supplemented by the protection offered by the elastomeric protecting element 54.

Furthermore, the provision of the elastomeric protecting element 54 permits not to have sharp edges at the protecting cup 52 or at the upper/lower frame members 20, 30, so as to offer a higher comfort to the user, in particular when the knee brace 10 is worn under the pants.

With reference to the enclosed figures, the knee brace 10 can be advantageously used by motorcyclists, particularly by motocross riders.

However, the knee brace 10 can also be used in other fields where an effective protection of the user's legs must be obtained. For example, the knee brace 10 can advantageously be worn by cyclists or skiers.

Moreover, the knee brace 10 can be an orthopedic device designed to control and limit the knee joint movements.

As above mentioned, the knee brace 10 comprises an upper frame member 20 which is designed for being fixed to the thigh of the user by means of securing means not shown in the enclosed figures.

The upper frame member 20 is preferably made of rigid material. Advantageously, the upper frame member 20 is made of polymeric rigid material. For example, the upper frame member 20 can be made of laminated carbon fibers.

Preferably, the inner portion of the upper frame member 20 is covered with a layer of soft material, not shown in the figures, so that the upper frame member 20 can be fastened in a comfortable way to the user's leg.

As shown for example in figures 2, 5, 6, 7, the upper frame member 20 is shaped so as to conform to the shape of the user's thigh.

Advantageously, the upper frame member 20 can be provided with a flexible wing 21 made of flexible polymeric material. The flexible wing 21 can be connected to the upper frame member 20 by means of known fastening means, for example rivets. The function of the flexible wing 21 is to provide customization to different tight circumferences.

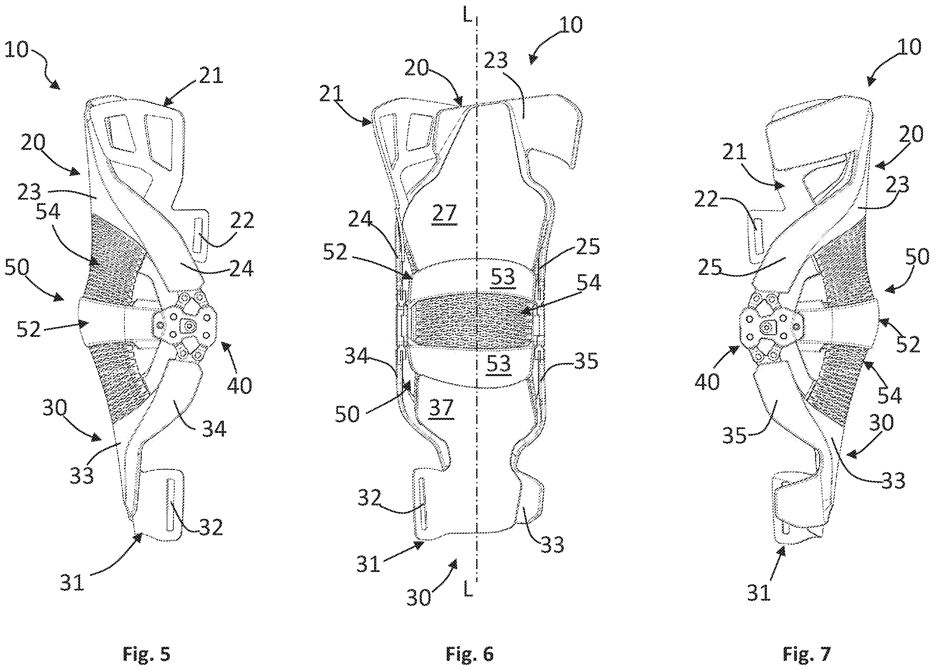

The flexible wing 21 can comprise a buckle 22 (see for example figures 5 and 7) suitable for being engaged by the securing means used for fixing the knee brace 10 to the user's leg.

Advantageously, the flexible wing 21 is suitable for evenly spreading over the thigh the tight force exerted by the securing means, for example straps, so as to avoid local pressures. In this way, the upper frame member 20 and thus the whole knee brace 10 are fixed in a stable way over the leg of the user, without affecting the comfort thereof.

The upper frame member 20 preferably comprises an upper main body 23, designed to be superimposed to the front portion of the thigh of the user's leg, an upper medial element 24, designed to be superimposed to the upper medial part of the knee joint, and an upper lateral element 25, designed to be superimposed to the upper lateral part of the knee joint.

Additional buckles 22 suitable for being engaged by the securing means used for fixing the knee brace to the user's leg can be provided on the upper main body 23 and on the upper medial element 24 (see for example figures 5 and 7).

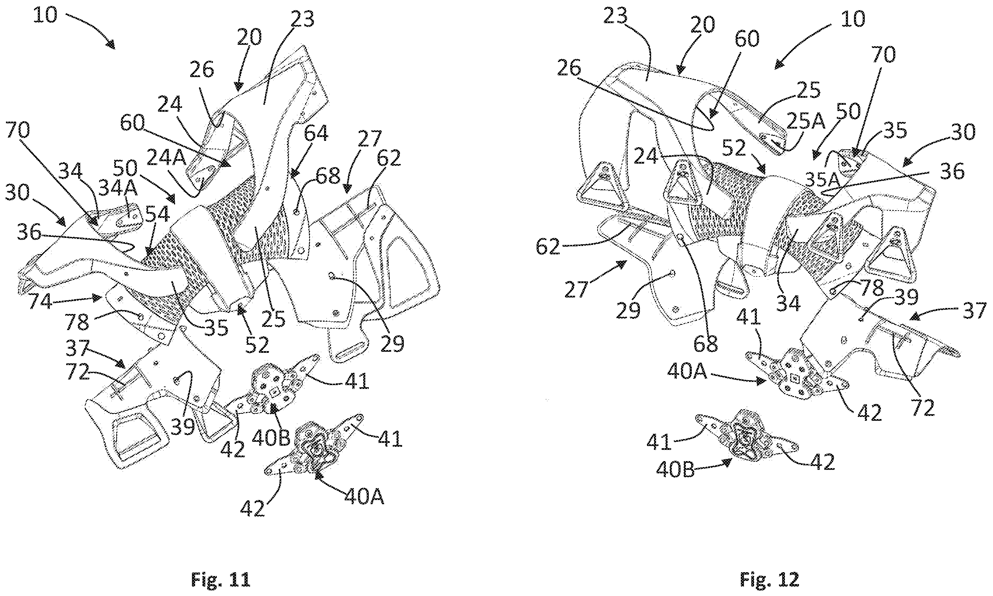

Preferably, upper medial element 24 and upper lateral element 25 are provided at their lower ends with seats 24A, 25A, each designed to be engaged by a corresponding mounting frame 41 of the associated articulation hinge 40 (see figures 11-12).

The upper main body 23 has a lower edge 26 which, together with the upper medial element 24 and the upper lateral element 25, delimits an upper space 60 designed to be at least partially occupied by the patella protecting device 50 (see for example figures 11-12).

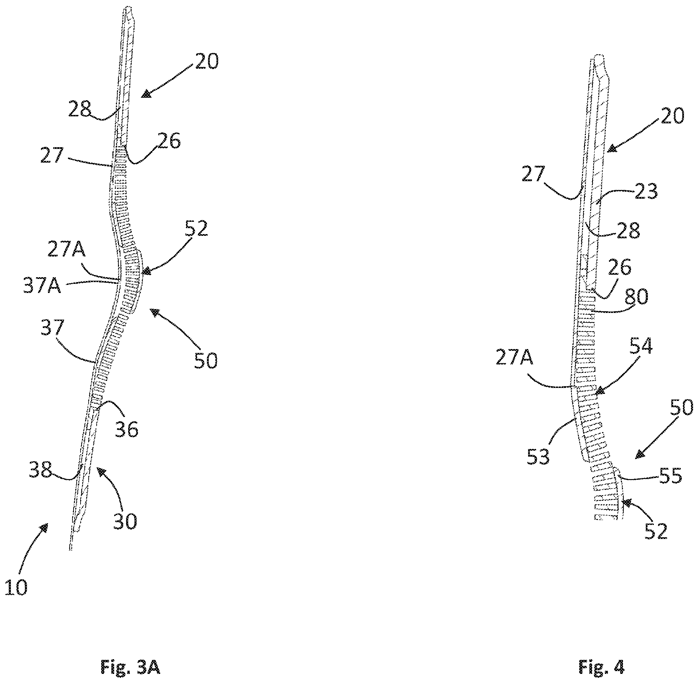

As clearly shown in figures 11-12, the upper frame member 20 preferably comprises an upper shield 27. Preferably, the upper shield 27 is coupled to the inner surface of the upper main body 23. In particular, the upper shield 27 is coupled to the inner surface of the upper main body 23 so as to create an interspace 28 (see for example figure 4) wherein, as it will be disclosed in detail in the following, an upper portion of the elastomeric protecting element 54 can be housed.

Preferably, the upper shield 27 is provided on its outer surface with spacer means 62 designed to keep spaced apart the upper shield 27 and the upper main body 23 so as to create the interspace 28.

Advantageously, the combination of the upper frame member 20 with the upper shield 27 improves the impact resistance properties of the knee brace at the thigh area.

In particular, the combination of the upper frame member 20 with the upper shield 27 allows to meet the safety requirements defined in the European standard EN 1621-1: 2012, which is specific for the motorcyclists' limb joint impact protectors, in a larger area than that requested by such a standard. Consequently, the knee brace of the present invention is able to offer an improved protection to the user.

Advantageously, the upper shield 27 can be made of semirigid material, namely a stiff material capable of resisting a deforming force and able to bend or be temporarily deformed by a force somewhat greater than said deforming force.

Preferably, the upper shield 27 is made of a semirigid polymeric material.

The lower portion of the upper shield 27 is provided with fastening means 29 (see for example figures 11-12) designed to block the elastomeric protecting element 54 inside the interspace 28.

Advantageously, if the knee brace is provided with an upper shield 27, the layer of soft material of the upper lower frame member 20 can be applied to the upper shield 27.

In detail, the inner surface of the upper shield 27 can be provided with further fastening means on which a padding layer can be attached.

In detail, the fastening means can consist of hook-and-loop fastening means. Preferably, the inner surface of the upper shield 27 can be provided with the hook side of the fastening means, while the padding layer to be attached to the upper shield 27 can be provided with the loop side of the fastening means.

The provision of the upper shield 27 permits to have a larger area provided with hook-and-loop fastening means, consequently an improved adhesion between the knee brace and the inner padding layer can be obtained, so as to offer an improved comfort to the user.

Preferably, the upper shield 27 extends from the upper frame member 20 towards the protecting cup 52.

Advantageously, when the knee brace is in the straight configuration (see for example figure 3), the lower edge 27A of the upper shield 27 is adjacent to the upper edge 52A of the protecting cup 52.

Alternatively, as shown in figure 3A, the lower edge 27A of the upper shield 27 can extend underneath the protecting cup 52

The lower frame member 30 is configured similar to the upper frame member 20.

As before mentioned, the lower frame member 30 is designed for being fixed to the shin of the user by means of securing means not shown in the enclosed figures.

The lower frame member 30 is preferably made of rigid material. Advantageously, the lower frame member 30 is made of polymeric rigid material. Similarly to the upper frame member 20, the lower frame member 30 can be made of laminated carbon fibers.

Preferably, the inner portion of the lower frame member 30 is covered with a layer of soft material, not shown in the figures, so that the lower frame member 30 can be fastened in a comfortable way to the user's leg.

As shown for example in figures 2, 5, 6, 7, the lower frame member 30 is shaped so as to conform to the shape of the user's shin.

The lower frame member 30 preferably comprises a lower main body 33, designed to be superimposed to the front portion of the shin of the user's leg, a lower medial element 34, designed to be superimposed to the lower medial part of the knee joint, and a lower lateral element 35, designed to be superimposed to the lower lateral part of the knee joint.

Lower medial element 34 and lower lateral element 25 are provided at their lower ends with seats 34A, 35A, each designed to be engaged by a corresponding mounting frame 42 of the associated articulation hinge 40 (see figures 11-12).

The lower main body 33 has an upper edge 36 which, together with the lower medial element 34 and the lower lateral element 35, delimits a lower space 70 designed to be at least partially occupied by the patella protecting device 50 (see for example figures 11-12).

As clearly shown in figures 11-12, similarly to the upper frame member 20, the lower frame member 30 preferably comprises a lower shield 37. Preferably, the lower shield 37 is coupled to the inner surface of the lower main body 33. In particular, the lower shield 37 is coupled to the inner surface of the lower main body 33 so as to create an interspace 38 (see for example figure 4) analogous to the interspace 28 formed between the upper main body 23 and the upper shield 27 and having the same function.

The lower shield 37 is provided on its outer surface with spacer means 72 designed to keep spaced apart the lower shield 37 and the lower main body 33 so as to create the interspace 38.

Advantageously, the combination of the lower frame member 30 with the lower shield 37 improves the impact resistance properties of the knee brace at the shin area. In particular, the combination of the lower frame member 30 with the lower shield 37, similarly to what mentioned before as regards the combination of the upper frame member 20 with the upper shield 27, allows the knee brace to meet the safety requirements defined in the European standard EN 1621-1: 2012 in a larger area than that requested by such a standard. Consequently, the knee brace of the present invention is able to offer an improved protection to the user at patella area, due to the presence of the patella protecting device, and at the upper area and at the lower area with respect to the patella, due to the combination of the upper frame member with the upper shield and the combination of the lower frame member with the lower shield.

Similarly to the upper shield 27, the lower shield 37 is preferably made of semirigid material.

Preferably, the lower shield 37 is made of a semirigid polymeric material.

The upper portion of the lower shield 37 is provided with fastening means 39 designed to block the elastomeric protecting element 54 inside the interspace 38.

Preferably, the lower shield 37 extends from the lower frame member 30 towards the protecting cup 52.

Advantageously, when the knee brace is in the straight configuration (see for example figure 3), the upper edge 37A of the lower shield 37 is adjacent to the lower edge 52B of the protecting cup 52.

Alternatively, as shown in figure 3A, the upper edge 37A of the lower shield 37 can extend underneath the protecting cup 52.

Preferably, the upper edge 37A extends underneath the protecting cup 52 towards the upper shield 27. More preferably, the upper edge 37A extends underneath the protecting cup 52 towards the upper shield 27 to be adjacent to the lower edge 27A of the upper shield 27 with the knee brace in the straight configuration.

Advantageously, if the knee brace is provided with a lower shield 37 the layer of soft material of the lower frame member 30 can be applied to the lower shield 37.

As a matter of fact, similarly to the inner surface of the upper shield 27, the inner surface of the lower shield 37 can be provided with further fastening means on which a padding layer can be attached.

In detail, the fastening means can consist of hook-and-loop fastening means. Preferably, the inner surface of the lower shield 37 can be provided with the hook side of the fastening means, while the padding layer to be attached to the lower shield 37 can be provided with the loop side of the fastening means.

As noted with reference to the upper shield 27, the provision of the lower shield 37 permits to have a larger area provided with hook-and-loop fastening means, consequently an improved adhesion between the knee brace and the inner padding layer can be obtained, so as to offer an improved comfort to the user.

This is particularly useful since the lower frame member 30 usually is worn under the boot. The boot fits tightly over the shin of the user and consequently high pressures can be exerted in that area. The provision of the lower shield 37 avoids that local pressures act on the shin of the user improving the comfort offered by the knee brace. The lower frame member 30 can be provided with a semirigid wing 31 designed to wrap the shin of the user so as to protect the side portions thereof. The semirigid wing 31 can be provided with a buckle 32 suitable for being engaged by the corresponding securing means used for fastening the knee brace 10 to the user's leg.

The semirigid wing 31 permits the lower frame member 30 to adapt to the shin's shape of the user. Moreover, the semirigid wing 31 from one side permits to improve the protection offered by the lower frame member 30 and on the other side it assures that in case of an impact there is no stiff edge pushing against the shin of the user.

As clearly visible in the enclosed figures, the upper frame 20 and the lower frame 30 are pivotally connected to each other by means of an articulation hinge 40.

Preferably, the knee brace 10 comprises two articulation hinges 40A, 40B. A medial articulation hinge 40A can pivotally connect the upper medial element 24 to the lower medial element 34 and a lateral articulation hinge 40B can pivotally connect the upper lateral element 25 to the lower lateral element 35.

The articulation hinges 40A, 40B are preferably polycentric hinges.

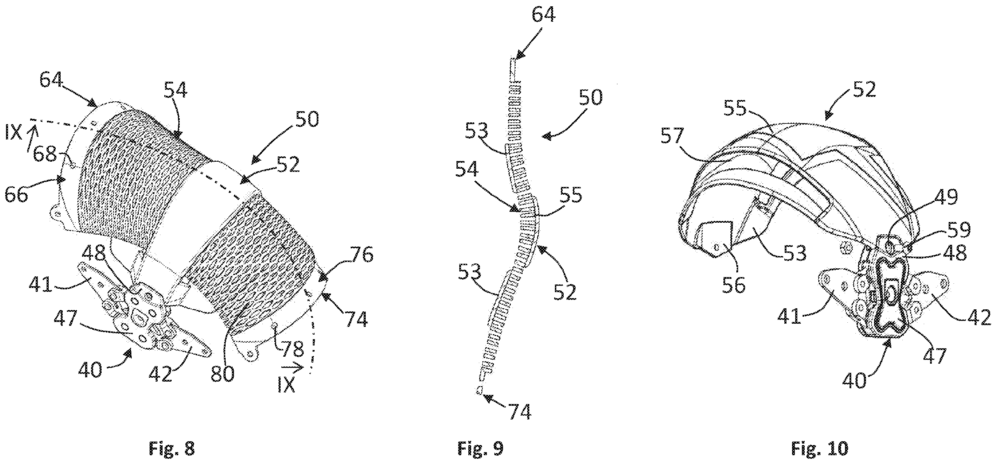

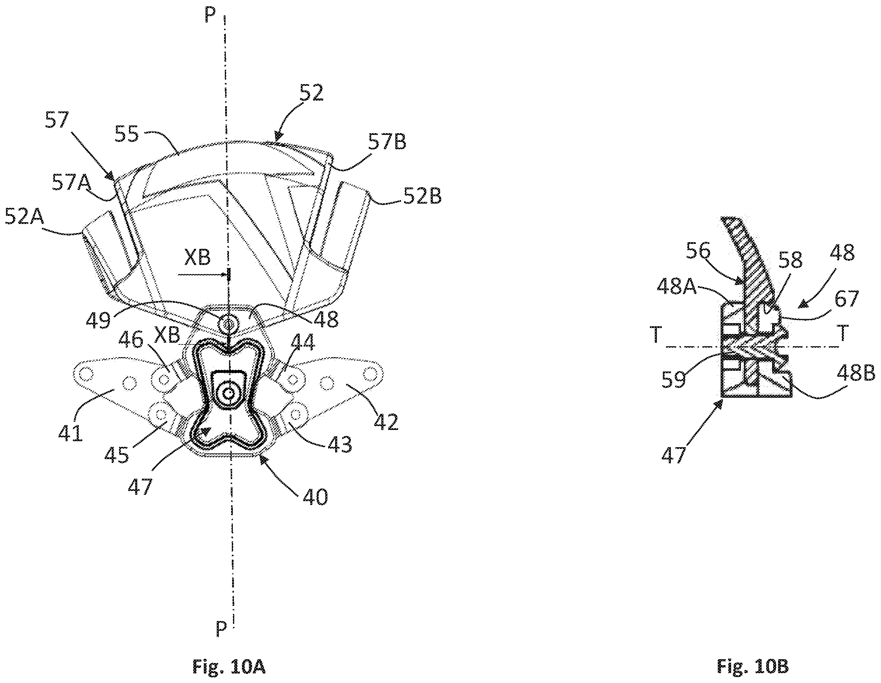

Each articulation hinge 40A, 40B can comprise a first couple of hinge arms 45, 46 and a second couple of hinge arms 43, 44 (see figure 10A).

Each hinge arm 45, 46 of the first couple and each hinge arm 43, 44 of the second couple has a first end pivotally enclosed inside a hinge body 47 of the articulation hinge 40 and a second end pivotally fixed to a corresponding mounting frame 41, 42.

In detail, the first couple of hinge arms 45, 46 is fixed to the mounting frame 41 designed to be connected to upper medial element 24 or to the upper lateral element 25, while the second couple of hinge arms 43, 44 is fixed to the mounting frame 42 designed to be connected to the lower medial element 34 or to the lower lateral element 35.

As shown in detail in figures 8, 10, 10A, 10B the hinge body 47 of each articulation hinge 40 can be provided with a fastening appendix 48.

Preferably, the fastening appendix 48 consists in a bearing fork formed by two spaced apart lugs 48A, 48B, each provided with a fastening through hole 49. Preferably, the fastening hole 49 is arranged along the longitudinal axis P of the hinge body 47 (see figure 10A).

The fastening appendix 48 is designed to accept a side appendix 56 of the protecting cup 52, which can be fixed therein by means of a fastening pin or rivet 59 engaging the fastening hole 49.

Consequently, as shown in the enclosed figures, the protecting cup 52 has a first side appendix 56 connected to the medial articulation hinge 40A and a second side appendix 56 connected to the lateral articulation hinge 40B.

Advantageously the appendix 56 is provided with an abutting surface 58 designed to get in contact with a corresponding abutting surface 67 of the appendix 48 to block the rotation of the protecting cup 52 around a transversal axis Z of the hinge, during the use of the knee brace (see figure 10B).

Consequently, the connection between the protecting cup 52 and the articulation hinges 40A, 40B is configured to maintain the protecting cup 52 correctly aligned over the patella of the user in all the different configurations of the knee brace.

Advantageously, the articulation hinge 40 can be of the type disclosed in the Italian patent application N° 102021000021527 in the name of the same applicant.

As before mentioned, the protecting cup 52 is part of the patella protecting device 50 which also comprises the elastomeric protecting element 54.

In detail, the protecting cup 52 and the elastomeric protecting element 54 are designed to cooperate with each other in order to protect the portion of the knee brace 10 delimited by the upper frame member 20 and the lower frame member 30.

In particular, the patella protecting device 50 is configured to occupy at least partially both the upper space 60, delimited by the lower edge 26, the upper medial element 24 and the upper lateral element 25, and the lower space 70, delimited by the upper edge 36, the lower medial element 34 and the lower lateral element 35.

The protecting cup 52 includes an inner surface 53 and an opposite outer surface 55. The inner surface 53 defines a generally concave surface, while the outer surface 55 defines a generally convex surface.

Preferably, the protecting cup 52 is made of rigid or semirigid material. Advantageously, the protecting cup 52 is made of a semirigid polymeric material.

According to the embodiment of figures 2-10, the protecting cup 52 is provided with a slot 57 inside which the elastomeric element 54 can be slidably arranged. Said slot 57, which is shown in detail in figures 10 and 10A, extends transversally with respect to the longitudinal axis L of the knee brace. As shown in figure 10A, the slot 57 has an upper opening 57A and a lower opening 57B. Consequently, the portion of the protecting cup 52 comprised between the upper opening 57A and the lower opening 57B of the slot 57 is arranged over the elastomeric protecting element 54, while the portions of the protecting cup 52 comprised between the upper edge 52A and the upper opening 57A and between the lower edge 52B and the lower opening 57B are arranged underneath the elastomeric protecting element 54.

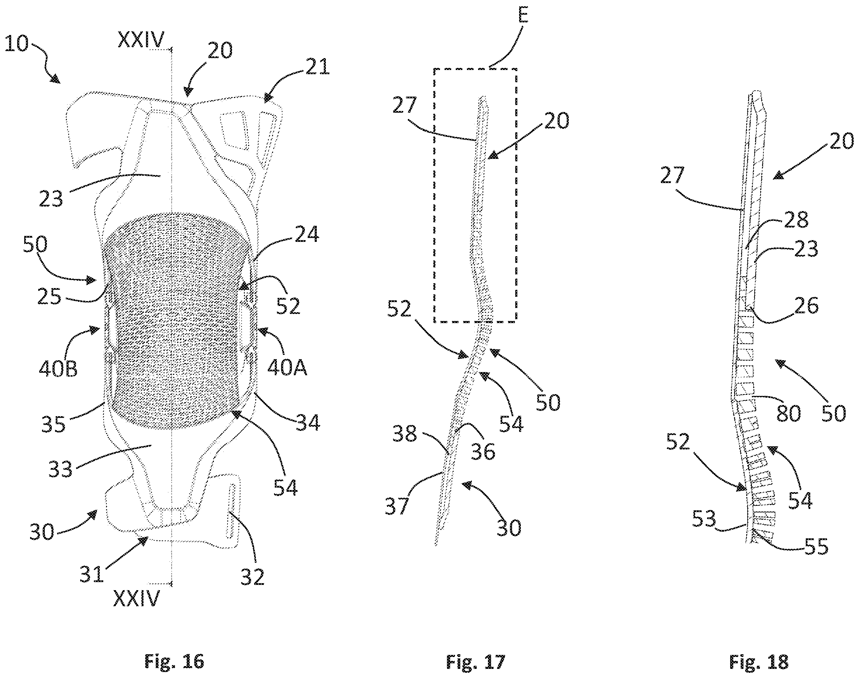

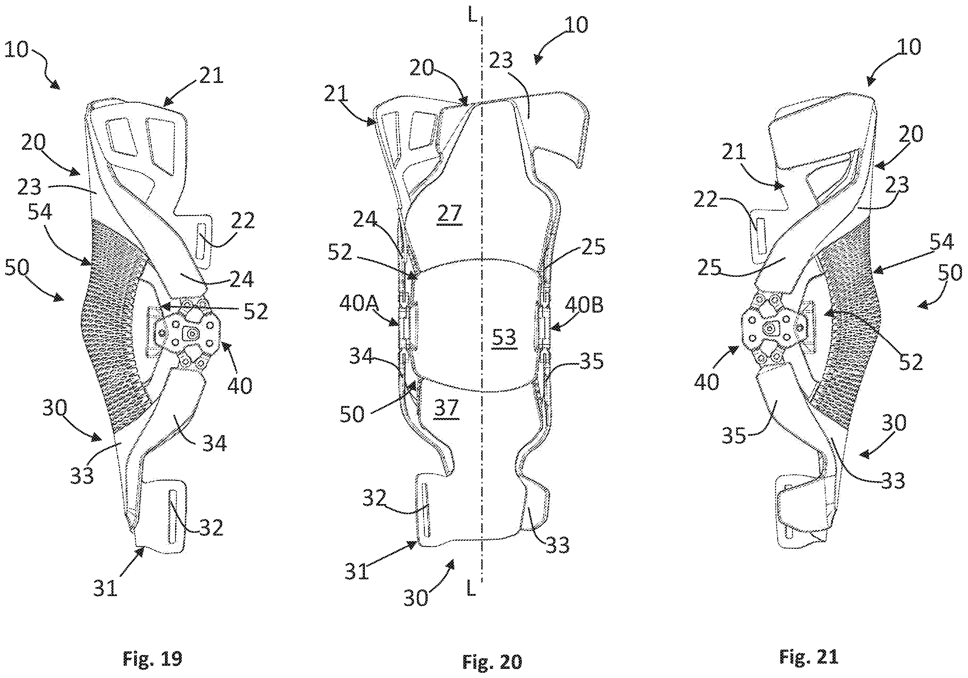

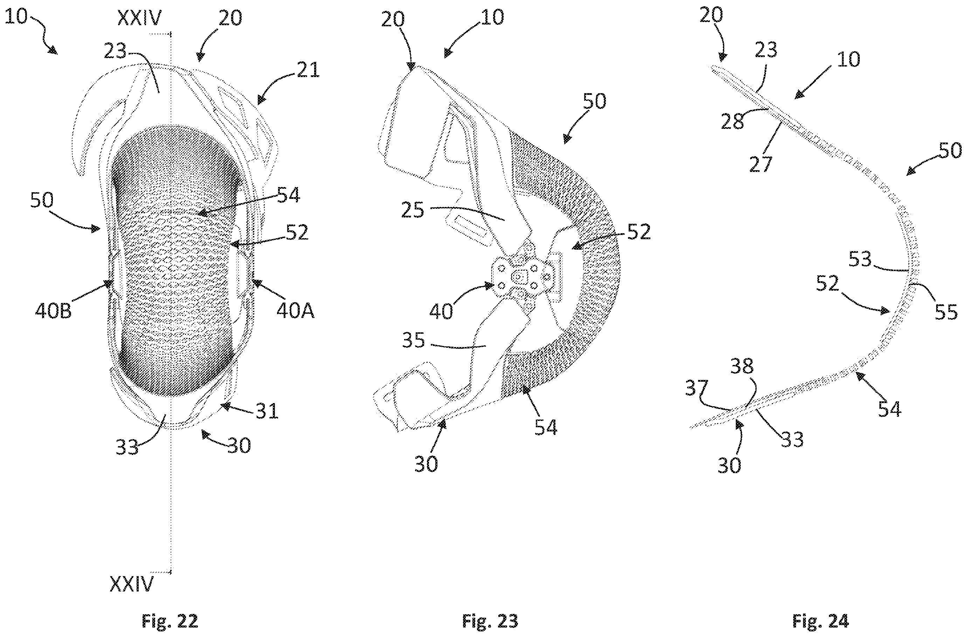

Alternatively, as shown in figures 16-24, the protecting cup 52 is designed to be arranged on the inner surface of the elastomeric protecting element 54, so as to be positioned underneath the protecting cup 52.

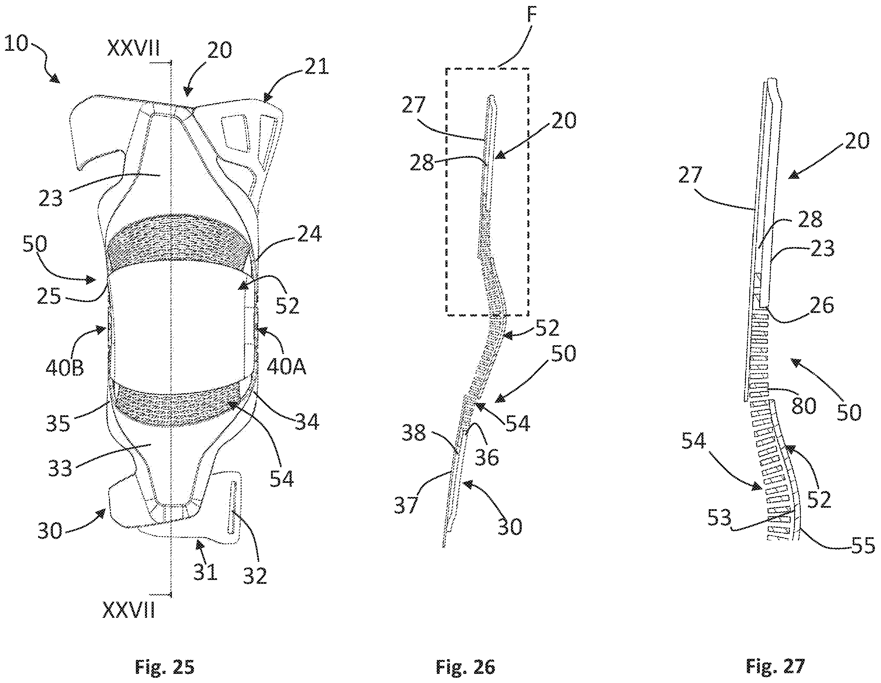

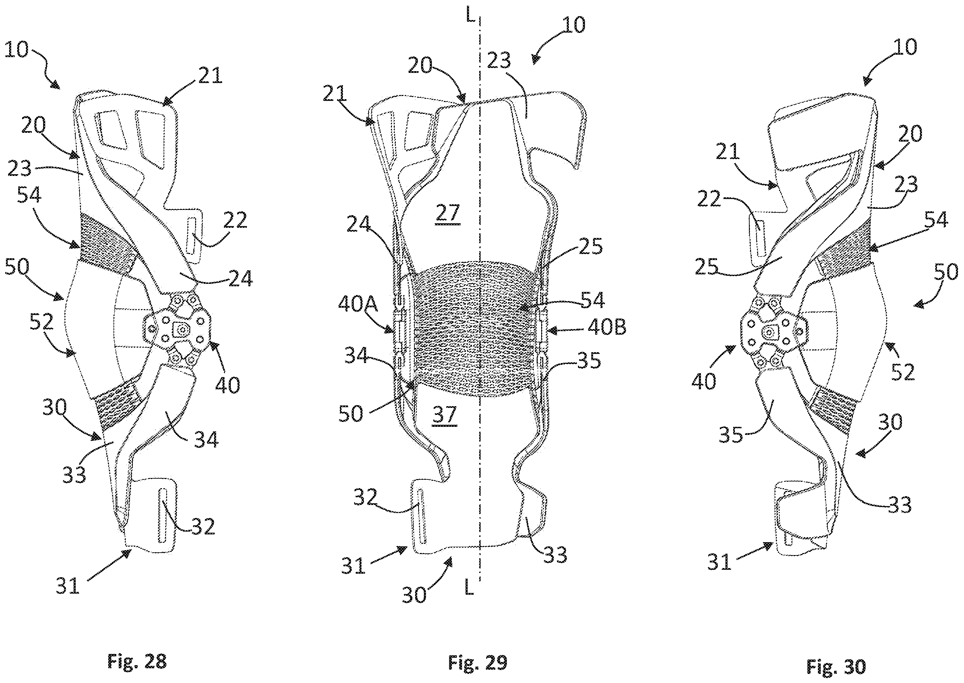

Moreover, in the embodiment shown in figures 25-30, the protecting cup 52 can be arranged on the outer surface of the elastomeric protecting element 54, so as to be positioned on top of the protecting cup 52.

In the embodiment of figures 16-24 and in the embodiment of figures 25-30, the protecting cup 52 can be provided with guiding means arranged respectively on the outer surface or on the inner surface (not shown in the attached figures) having the function to maintain the proper alignment between the elastomeric protecting element 54 and the protecting cup 52.

The elastomeric protecting element 54 in turn is designed to extend from the upper frame member 20 to the lower frame member 30.

In the enclosed figures, a single elastomeric protecting element 54 is shown. However, the elastomeric protecting element 54 can be formed by two or more elements fastened to each other.

Alternatively, a first element can extend from the upper frame member to the protecting cup and a second element can extend from the lower frame member to the protecting cup. Also in this case, in the meaning of the present invention, the elastomeric protecting element 54 is considered to extend from the upper frame member to the lower frame member.

According to the invention, the elastomeric protecting element 54 is directly or indirectly fastened to the upper frame member 20 and to the lower frame member 30.

The elastomeric protecting element 54 has an upper end 64 and a lower end 74.

The upper end 64 of the elastomeric protecting element 54 is designed to be fastened to the inner surface of the upper frame member 20. Preferably, the upper end 64 is fastened to the inner surface of the upper main body 23. Advantageously, as before mentioned, the upper end 64 can be blocked inside the interspace 28 formed between the upper main body 23 and the upper shield 27.

Similarly, the lower end 74 of the elastomeric protecting element 54 is designed to be fastened to the inner surface of the lower frame member 30. Preferably, the lower end 74 is fastened to the inner surface of the lower main body 33. Advantageously, as before mentioned, the lower end 74 can be blocked inside the interspace 38 formed between the lower main body 33 and the lower shield 37.

As clearly shown in figure 8, the elastomeric protecting element 54 can be provided at the upper end 64 and at the lower end 74 with corresponding retaining edges 66, 76. Said retaining edges 66, 76 are preferably provided with fastening holes 68, 78 designed to respectively engage the fastening means 29 provided on the upper shield 27 and the fastening means 39 provided on the lower shield 37. Known fastening means, like for example rivets, can be used to block the elastomeric protecting element 54 to the upper and lower shields 27, 37.

Advantageously, the elastomeric protecting element 54 is made of an elastic polymeric material. Preferably, the elastomeric protecting element 54 is made of an extensible thermoplastic compound.

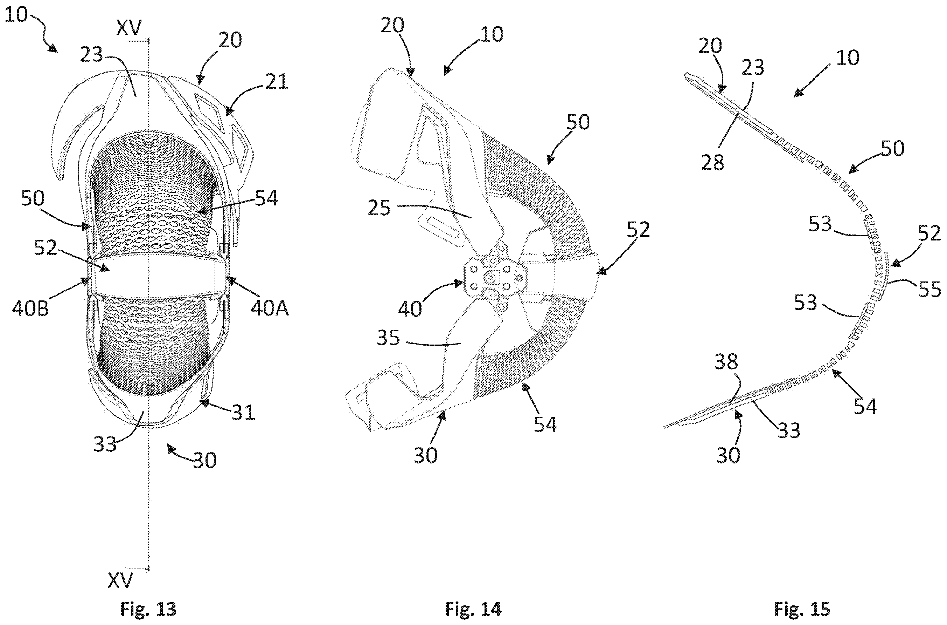

In this way, the elastomeric protecting element 54 is able to be stretched along the longitudinal axis L of the knee brace 10 when the latter moves from a straight configuration, shown for example in figures 2-7, to a bent configuration, shown for example in figures 13-15.

Advantageously, the elastomeric protecting element 54 can have a mesh structure which substantially extends uniformly over the whole surface of the elastomeric protecting element 54, with the exception of the retaining edges 66, 76, if present.

The mesh structure of the elastomeric protecting element 54 is helpful in increasing the stretchability of the element itself. At the same time, thanks to the provision of the mesh structure the protecting element 54 permits the passage of air so as to improve the breathability of the whole knee brace.

As shown in the enclosed figures, the mesh structure of the protecting element 54 defines a plurality of openings 80.

Preferably the openings 80 have an elongated shape. More preferably, the elongated dimension is arranged perpendicularly to the longitudinal axis L of the knee brace.

This specific design allows the elastomeric protecting element 54 to be elongated during the passage from the straight configuration to a bent configuration without opposing such a movement.

At the same time, being made of a stretchable material, the elastomeric protecting element 54 helps the knee brace 10 to return from a bent configuration into a straight configuration, being helpful in reducing the fatigue of the rider.

Moreover, the mutual arrangement between the protecting cup 52 and the elastomeric protecting element 54 assures that the latter can be freely stretched so as to follow the movements of the knee.

As a matter of fact, the elastomeric protecting element 54 can move independently from the protecting cup 52.

The thickness of the elastomeric protecting element 54 is selected according to the impact resistance that it is desired to be obtained. The thickness can be constant or can gradually decrease from the central portion towards the upper and lower ends.

In the embodiment of figures 2-4, the elastomeric protecting element 54 has a thickness which is lower than the height of the slot 57 so as to be able to freely slide inside the slot, in both directions, namely the extension and the compression directions

Advantageously, the elastomeric protecting element 54 has a width which substantially corresponds to the width of the protecting cup 52. Consequently, when the elastomeric protecting element 54 is arranged over the protecting cup 52, the latter, with the exception of the side appendixes 56, is covered by the elastomeric protecting element 54.

Similarly, in the embodiment wherein the elastomeric protecting element 54 is slidably arranged inside the slot 57 of the protecting cup 52, the width of the elastomeric protecting element will be substantially equal to the width of the slot 57. In the preferred embodiment, such a slot 57 extends from one side to the other of the protecting cup 52.

It is thus clear how the predefined objects may be achieved with the knee brace 10 according to the invention.

As a matter of fact, the combination of the elastomeric protecting element 54 with the protecting cup 52 provides an improved configuration to patella and leg of the user in all operative configurations.

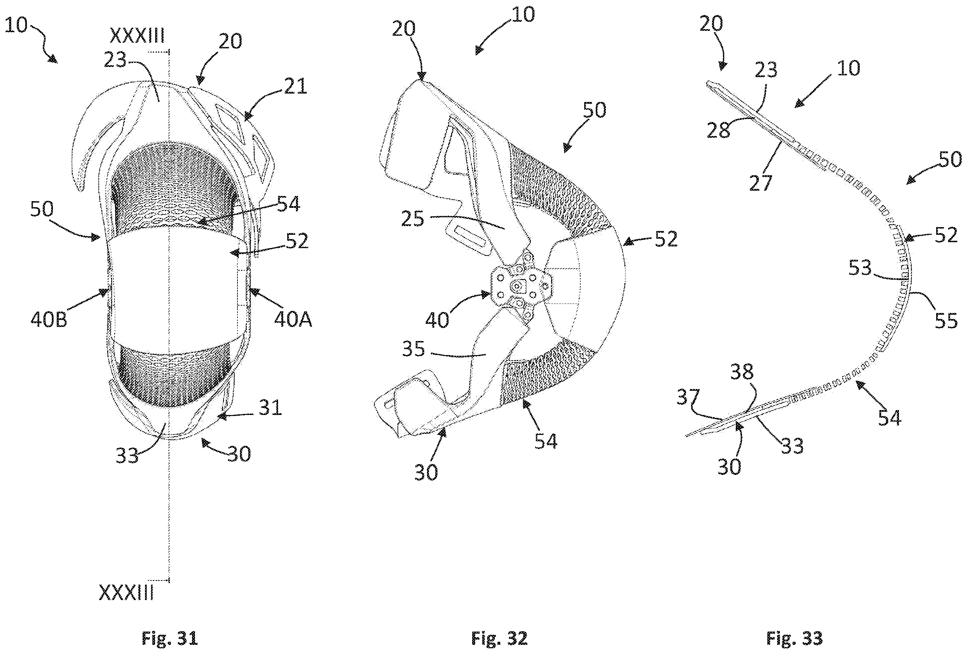

As a matter of fact, as shown in figures 13-15, 22-24, 31-33, even in a bent configuration of the knee brace, the elastomeric protecting element 54 is able to offer adequate protection to the underlying portion of the user's leg.

In particular, the elastomeric protecting element 54 is able to fill the gaps between the upper and lower frame members and the protecting cup, so as to protect, in combination with the upper and lower shields, if present, also the area external to the protecting cup 52. In detail, the knee joint is more protected against front and side impacts.

At the same time, even in the straight configuration of the knee brace, see for example figures 2-4, 16-18, 25-27, the combination of the elastomeric protecting element 54 with the protecting cup 52 offers an improved protection, since the protection of the protecting cup 52 is supplemented by the elastomeric protecting element 54. The two layers assembly formed by the protecting cup 52 and the elastomeric protecting element 54 from one side is able to resist to impacts having a greater intensity with respect to a single layer protection, from the other side, thanks to the provision of the elastomeric protecting element 54, is also able to elastically deform so as to provide an efficient absorption of the strikes acting against the knee brace.

Furthermore, in the embodiments of the knee brace provided with upper and lower shields, the knee brace offers an improved protection to the whole user's leg and not only to the patella area. As a matter of fact, by making reference for example to figures 3, 3A, 4, 18, 27, in the area external to the protecting cup, the knee brace equally offers a double layers protection: the first layer being formed by the elastomeric protecting element 54 and the second layer being formed by the upper or lower shield. This combination of the elastomeric protecting element 54 with the upper/lower shield 27, 37 is particularly useful when the knee brace is in the straight configuration. However, also in the bent configuration of the knee brace, the combination of the elastomeric protecting element 54 with the upper/lower shield 27, 37 is suitable to offer an improved protection to the leg of the user.

Again, the elastomeric protecting element 54 being made of stretchable material is able to better adapt itself to the leg of the user, following the movements thereof and offering at the same time an improved protection.

Furthermore, the mesh structure of the elastomeric protecting element 54 is able to offer improved ventilation to the user's leg during the use, assuring at the same time a high stability of the knee brace.

Finally, in all the embodiments above disclosed, the provision of the elastomeric protecting element 54 permits not to have sharp edges at the perimeter portion of the upper/lower frames and/or at the protecting cup. As shown in the enclosed figures, both in the straight and bent configuration, the elastomeric protection element 54 is able to fill the edges of the upper/lower frames and protecting cup so as to give to the knee brace a smooth out looking aspect.

With regard to the embodiments of the knee brace 10 described above, the person skilled in the art may, in order to satisfy specific requirements, make modifications to and/or replace elements described with equivalent elements, without thereby departing from the scope of the accompanying claims.

## Claims

1. A knee brace (10) comprising:
- an upper frame member (20), designed for being fastened to the upper part of the user's leg, and a lower frame member (30), designed for being fastened to the lower part of the user's leg; the upper frame member (20) and the lower frame member (30) being pivotally connected to each other by means of at least one articulation hinge (40);
- a patella protecting device (50) positioned between the upper frame member (20) and the lower frame member (30); the patella protecting device (50) comprising a protecting cup (52) coupled to said at least one articulation hinge (40) and designed to be positioned over the patella of the user;
**characterized in that** the patella protecting device (50) further comprises an elastomeric protecting element (54) extending between the upper frame member (20) and the lower frame member (30) and operatively coupled to the protecting cup (52) and wherein the elastomeric protecting element (54) is directly or indirectly fastened to the upper frame member (20) and to the lower frame member (30).

2. Knee brace (10) according to claim 1, **characterized in that** the elastomeric protecting element (54) has an upper end (64) and a lower end (74); the upper end (64) being fastened to an inner surface of the upper frame member (20) and the lower end (74) being fastened to an inner surface of the lower frame member (30).

3. Knee brace (10) according to any one of the preceding claims, **characterized in that** the upper frame member (20) comprises an upper main body (23), designed to be superimposed to the front portion of the thigh of the user's leg, an upper medial element (24), designed to be superimposed to the upper medial part of the knee joint, and an upper lateral element (25), designed to be superimposed to the upper lateral part of the knee joint.

4. Knee brace (10) according to any one of the preceding claims, **characterized in that** the lower frame member (30) comprises a lower main body (33), designed to be superimposed to the front portion of the shin of the user's leg, a lower medial element (34), designed to be superimposed to the lower medial part of the knee joint, and a lower lateral element (35), designed to be superimposed to the lower lateral part of the knee joint.

5. Knee brace (10) according to any one of the preceding claims, **characterized in that** the upper frame member (20) comprises an upper shield (27) and the lower frame member (30) comprises a lower shield (37); the upper shield (27) being coupled to an inner surface of the upper main body (23) and the lower shield (37) being coupled to an inner surface of the lower main body (33).

6. Knee brace (10) according to any one of the preceding claims, **characterized in that** the upper end (64) of the elastomeric protecting element (54) is blocked inside an interspace (28) formed between the upper main body (23) and the upper shield (27) and the lower end (74) is blocked inside an interspace (38) formed between the lower main body (33) and the lower shield (37).

7. Knee brace (10) according to any one of the preceding claims, **characterized in that** the protecting cup (52) is made of rigid or semirigid material and the elastomeric protecting element (54) is made of an elastic polymeric material.

8. Knee brace (10) according to any one of the preceding claims, **characterized in that** the elastomeric protecting element (54) has a mesh structure extending uniformly over the whole surface thereof.

9. Knee brace (10) according to claim 8, **characterized in that** said mesh structure defines a plurality of openings (80); each opening of said plurality having an elongated shape.

10. Knee brace (10) according to claim 9, **characterized in that** the elongated dimension of each opening (80) of said plurality is arranged perpendicularly to a longitudinal axis (L) of the knee brace (10).

11. Knee brace (10) according to any one of the preceding claims, **characterized by** comprising two articulation hinges (40A, 40B); a medial articulation hinge (40A) pivotally connecting the upper medial element (24) to the lower medial element (34) and a lateral articulation hinge (40B) pivotally connecting the upper lateral element (25) to the lower lateral element (35); the protecting cup (52) having a first side appendix (56) connected to the medial articulation hinge (40A) and a second side appendix (56) connected to the lateral articulation hinge (40B).

12. Knee brace (10) according to claim 11, **characterized in that** each side appendix (56) of the protecting cup (52) is provided with an abutting surface (58) designed to get in contact with a corresponding abutting surface (67) of an appendix (48) of an hinge body (47) of the articulation hinge (40A, 40B) to block a rotation of the protecting cup (52) around a transversal axis (Z) of the articulation hinge (40A, 40B).

13. Knee brace (10) according to claim 5, **characterized in that**, with the knee brace (10) in a straight configuration, a lower edge (27A) of the upper shield (27) is adjacent to an upper edge (52A) of the protecting cup (52) and an upper edge (37A) of the lower shield (37) is adjacent to a lower edge (52B) of the protecting cup (52).

14. Knee brace (10) according to claim 5, **characterized in that** the upper shield (27) and the lower shield (37) extends from the upper frame member (20) and the lower frame member (30) underneath the protecting cup (52), so that an upper edge (37A) of the lower shield (37) is adjacent to a lower edge (27A) of the upper shield (27) with the knee brace in the straight configuration.

15. Knee brace (10) according to any one of the preceding claims, **characterized in that** the protecting cup (52) is provided with a slot (57) inside which the elastomeric protecting element (54) is slidably arranged.

16. Knee brace (10) according to claim 1, **characterized in that** the protecting cup (52) is arranged on the inner surface of the elastomeric protecting element (54).

17. Knee brace (10) according to claim 1, **characterized in that** the protecting cup (52) is arranged on the outer surface of the elastomeric protecting element (54).

## Patentansprüche

1. Knieschiene (10), umfassend:
- ein oberes Rahmenelement (20), das zum Befestigen am oberen Teil des Beins des Benutzers ausgelegt ist, und ein unteres Rahmenelement (30), das zum Befestigen am unteren Teil des Beins des Benutzers ausgelegt ist; wobei das obere Rahmenelement (20) und das untere Rahmenelement (30) durch mindestens ein Gelenkscharnier (40) schwenkbar miteinander verbunden sind;
- eine Kniescheibenschutzvorrichtung (50), die zwischen dem oberen Rahmenelement (20) und dem unteren Rahmenelement (30) angeordnet ist; wobei die Kniescheibenschutzvorrichtung (50) eine Schutzschale (52) umfasst, die mit dem mindestens einen Gelenkscharnier (40) verbunden ist und zur Anordnung über der Kniescheibe des Benutzers ausgelegt ist;
**dadurch gekennzeichnet, dass** die Kniescheibenschutzvorrichtung (50) weiter ein Elastomer-Schutzelement (54) umfasst, das sich zwischen dem oberen Rahmenelement (20) und dem unteren Rahmenelement (30) erstreckt und funktional mit der Schutzschale (52) verbunden ist, und wobei das Elastomer-Schutzelement (54) direkt oder indirekt mit dem oberen Rahmenelement (20) und dem unteren Rahmenelement (30) verbunden ist.

2. Knieschiene (10) gemäß Anspruch 1, **dadurch gekennzeichnet, dass** das Elastomer-Schutzelement (54) ein oberes Ende (64) und ein unteres Ende (74) aufweist; wobei das obere Ende (64) an einer Innenfläche des oberen Rahmenelements (20) befestigt ist und das untere Ende (74) an einer Innenfläche des unteren Rahmenelements (30) befestigt ist.

3. Knieschiene (10) gemäß einem der vorhergehenden Ansprüche, **dadurch gekennzeichnet, dass** das obere Rahmenelement (20) einen oberen Hauptkörper (23) umfasst, der zum Überlagern des vorderen Abschnitts des Oberschenkels des Beins des Benutzers ausgelegt ist, ein oberes Medialelement (24), das zum Überlagern des oberen medialen Teils des Kniegelenks ausgelegt ist, und ein oberes Seitenelement (25), das zum Überlagern des oberen seitlichen Teils des Kniegelenks ausgelegt ist.

4. Knieschiene (10) gemäß einem der vorhergehenden Ansprüche, **dadurch gekennzeichnet, dass** das untere Rahmenelement (30) einen unteren Hauptkörper (33) umfasst, der zum Überlagern des vorderen Abschnitts des Schienbeins des Beins des Benutzers ausgelegt ist, ein unteres Medialelement (34), das zum Überlagern des unteren medialen Teils des Kniegelenks ausgelegt ist, und ein unteres Seitenelement (35), das zum Überlagern des unteren seitlichen Teils des Kniegelenks ausgelegt ist.

5. Knieschiene (10) gemäß einem der vorhergehenden Ansprüche, **dadurch gekennzeichnet, dass** das obere Rahmenelement (20) ein oberes Schild (27) umfasst und das untere Rahmenelement (30) ein unteres Schild (37) umfasst; wobei das obere Schild (27) mit einer Innenfläche des oberen Hauptkörpers (23) verbunden ist und das untere Schild (37) mit einer Innenfläche des unteren Hauptkörpers (33) verbunden ist.

6. Knieschiene (10) gemäß einem der vorhergehenden Ansprüche, **dadurch gekennzeichnet, dass** das obere Ende (64) des Elastomer-Schutzelements (54) in einem Zwischenraum (28) blockiert ist, der zwischen dem oberen Hauptkörper (23) und dem oberen Schild (27) gebildet ist, und das untere Ende (74) in einem Zwischenraum (38) blockiert ist, der zwischen dem unteren Hauptkörper (33) und dem unteren Schild (37) gebildet ist.

7. Knieschiene (10) gemäß einem der vorhergehenden Ansprüche, **dadurch gekennzeichnet, dass** die Schutzschale (52) aus starrem oder halbstarrem Material besteht und das Elastomer-Schutzelement (54) aus einem elastischen Polymermaterial besteht.

8. Knieschiene (10) gemäß einem der vorhergehenden Ansprüche, **dadurch gekennzeichnet, dass** das Elastomer-Schutzelement (54) eine Maschenstruktur aufweist, die sich gleichmäßig über dessen gesamte Oberfläche erstreckt.

9. Knieschiene (10) gemäß Anspruch 8, **dadurch gekennzeichnet, dass** die Maschenstruktur eine Mehrzahl von Öffnungen (80) definiert; wobei jede der Mehrzahl der Öffnungen eine längliche Form aufweist.

10. Knieschiene (10) gemäß Anspruch 9, **dadurch gekennzeichnet, dass** die Längsabmessung jeder der Mehrzahl der Öffnungen (80) senkrecht zu einer Längsachse (L) der Knieschiene (10) angeordnet ist.

11. Knieschiene (10) gemäß einem der vorhergehenden Ansprüche, **dadurch gekennzeichnet, dass** sie zwei Gelenkscharniere (40A, 40B) umfasst; wobei ein mediales Gelenkscharnier (40A) das obere Medialelement (24) mit dem unteren Medialelement (34) schwenkbar verbindet, und ein seitliches Gelenkscharnier (40B) das obere Seitenelement (25) mit dem unteren Seitenelement (35) schwenkbar verbindet; die Schutzschale (52) einen ersten Seitenfortsatz (56), der mit dem medialen Gelenkscharnier (40A) verbunden ist, und einen zweiten Seitenfortsatz (56), der mit dem seitlichen Gelenkscharnier (40B) verbunden ist, aufweist.

12. Knieschiene (10) gemäß Anspruch 11, **dadurch gekennzeichnet, dass** jeder Seitenfortsatz (56) der Schutzschale (52) mit einer Anschlagfläche (58) versehen ist, die zum in-Kontakt-Kommen mit einer entsprechenden Anschlagfläche (67) eines Fortsatzes (48) eines Scharnierkörpers (47) des Gelenkscharniers (40A, 40B) ausgelegt ist, um eine Drehung der Schutzschale (52) um eine Querachse (Z) des Gelenkscharniers (40A, 40B) zu blockieren.

13. Knieschiene (10) gemäß Anspruch 5, **dadurch gekennzeichnet, dass**, wenn die Knieschiene (10) in einer geraden Konfiguration ist, eine untere Kante (27A) des oberen Schilds (27) an eine obere Kante (52A) der Schutzschale (52) angrenzt und eine obere Kante (37A) des unteren Schilds (37) an eine untere Kante (52B) der Schutzschale (52) angrenzt.

14. Knieschiene (10) gemäß Anspruch 5, **dadurch gekennzeichnet, dass** sich das obere Schild (27) und das untere Schild (37) von dem oberen Rahmenelement (20) und dem unteren Rahmenelement (30) unter der Schutzschale (52) erstrecken, so dass eine obere Kante (37A) des unteren Schilds (37) an eine untere Kante (27A) des oberen Schilds (27) angrenzt, wenn die Knieschiene in der geraden Konfiguration ist.

15. Knieschiene (10) gemäß einem der vorhergehenden Ansprüche, **dadurch gekennzeichnet, dass** die Schutzschale (52) mit einem Schlitz (57) versehen ist, in dem das Elastomer-Schutzelement (54) verschiebbar angeordnet ist.

16. Knieschiene (10) gemäß Anspruch 1, **dadurch gekennzeichnet, dass** die Schutzschale (52) auf der Innenfläche des Elastomer-Schutzelements (54) angeordnet ist.

17. Knieschiene (10) gemäß Anspruch 1, **dadurch gekennzeichnet, dass** die Schutzschale (52) auf der Außenfläche des Elastomer-Schutzelements (54) angeordnet ist.

## Revendications

1. Orthèse de genou (10) comprenant:
- un élément de cadre supérieur (20), conçu pour être fixé à la partie supérieure de la jambe de l'utilisateur, et un élément de cadre inférieur (30), conçu pour être fixé à la partie inférieure de la jambe de l'utilisateur ; l'élément de cadre supérieur (20) et l'élément de cadre inférieur (30) étant reliés de manière pivotante l'un à l'autre au moyen d'au moins une charnière d'articulation (40);
- un dispositif de protection de la rotule (50) positionné entre l'élément de cadre supérieur (20) et l'élément de cadre inférieur (30) ; le dispositif de protection de la rotule (50) comprenant une coupelle de protection (52) couplée audit au moins une charnière d'articulation (40) et conçue pour être positionnée au-dessus de la rotule de l'utilisateur;
**caractérisé en ce que** le dispositif de protection de la rotule (50) comprend en outre un élément de protection élastomère (54) qui s'étend entre l'élément de cadre supérieur (20) et l'élément de cadre inférieur (30) et qui est couplé de manière fonctionnelle à la coupelle de protection (52) et lequel élément de protection élastomère (54) est fixé directement ou indirectement à l'élément de cadre supérieur (20) et à l'élément de cadre inférieur (30).

2. Orthèse de genou (10) selon la revendication 1, **caractérisée en ce que** l'élément de protection élastomère (54) présente une extrémité supérieure (64) et une extrémité inférieure (74); l'extrémité supérieure (64) étant fixée à une surface interne de l'élément de cadre supérieur (20) et l'extrémité inférieure (74) étant fixée à une surface interne de l'élément de cadre inférieur (30).

3. Orthèse de genou (10) selon l'une quelconque des revendications précédentes, **caractérisée en ce que** l'élément de cadre supérieur (20) comprend un corps principal supérieur (23), conçu pour être superposé à la partie avant de la cuisse de la jambe de l'utilisateur, un élément médial supérieur (24), conçu pour être superposé à la partie médiale supérieure de l'articulation du genou, et un élément latéral supérieur (25), conçu pour être superposé à la partie latérale supérieure de l'articulation du genou.

4. Orthèse de genou (10) selon l'une quelconque des revendications précédentes, **caractérisée en ce que** l'élément de cadre inférieur (30) comprend un corps principal inférieur (33), conçu pour être superposé à la partie avant du tibia de la jambe de l'utilisateur, un élément médial inférieur (34), conçu pour être superposé à la partie médiale inférieure de l'articulation du genou, et un élément latéral inférieur (35), conçu pour être superposé à la partie latérale inférieure de l'articulation du genou.

5. Orthèse de genou (10) selon l'une quelconque des revendications précédentes, **caractérisée en ce que** l'élément de cadre supérieur (20) comprend un écran supérieur (27) et l'élément de cadre inférieur (30) comprend un écran inférieur (37) ; l'écran supérieur (27) étant couplé à une surface interne du corps principal supérieur (23) et l'écran inférieur (37) étant couplé à une surface interne du corps principal inférieur (33).

6. Orthèse de genou (10) selon l'une quelconque des revendications précédentes, **caractérisée en ce que** l'extrémité supérieure (64) de l'élément de protection élastomère (54) est bloquée à l'intérieur d'un espace intermédiaire (28) formé entre le corps principal supérieur (23) et l'écran supérieur (27) et l'extrémité inférieure (74) est bloquée à l'intérieur d'un espace intermédiaire (38) formé entre le corps principal inférieur (33) et l'écran inférieur (37).

7. Orthèse de genou (10) selon l'une quelconque des revendications précédentes, **caractérisée en ce que** la coupelle de protection (52) est réalisée en matériau rigide ou en matériau semi-rigide et l'élément de protection élastomère (54) est réalisé en matériau polymère élastique.

8. Orthèse de genou (10) selon l'une quelconque des revendications précédentes, **caractérisée en ce que** l'élément de protection élastomère (54) présente une structure en treillis qui s'étend uniformément sur toute sa surface.

9. Orthèse de genou (10) selon la revendication 8, **caractérisée en ce que** ladite structure en treillis définit une pluralité d'ouvertures (80); chaque ouverture de ladite pluralité ayant une forme allongée.

10. Orthèse de genou (10) selon la revendication 9, **caractérisée en ce que** la dimension allongée de chaque ouverture (80) de ladite pluralité est disposée perpendiculairement à un axe longitudinal (L) de l'orthèse de genou (10).

11. Orthèse de genou (10) selon l'une quelconque des revendications précédentes, **caractérisée par le fait qu'**elle comprend deux charnières d'articulation (40A, 40B); une charnière d'articulation médiale (40A) reliant de manière pivotante l'élément médial supérieur (24) à l'élément médial inférieur (34) et une charnière d'articulation latérale (40B) reliant de manière pivotante l'élément latéral supérieur (25) à l'élément latéral inférieur (35) ; la coupelle de protection (52) présentant un premier appendice latéral (56) relié à la charnière d'articulation médiale (40A) et un second appendice latéral (56) relié à la charnière d'articulation latérale (40B).

12. Orthèse de genou (10) selon la revendication 11, **caractérisée en ce que** chaque appendice latéral (56) de la coupelle de protection (52) est pourvu d'une surface d'appui (58) conçue pour entrer en contact avec une surface d'appui correspondante (67) d'un appendice (48) d'un corps de charnière (47) de la charnière d'articulation (40A, 40B) pour bloquer une rotation de la coupelle de protection (52) autour d'un axe transversal (Z) de la charnière d'articulation (40A, 40B).

13. Orthèse de genou (10) selon la revendication 5, **caractérisée en ce que**, lorsque l'orthèse de genou (10) est dans une configuration droite, un bord inférieur (27A) de l'écran supérieur (27) est adjacent à un bord supérieur (52A) de la coupelle de protection (52) et un bord supérieur (37A) de l'écran inférieur (37) est adjacent à un bord inférieur (52B) de la coupelle de protection (52).

14. Orthèse de genou (10) selon la revendication 5, **caractérisée en ce que** l'écran supérieur (27) et l'écran inférieur (37) s'étendent à partir de l'élément de cadre supérieur (20) et de l'élément de cadre inférieur (30) sous la coupelle de protection (52), de sorte qu'un bord supérieur (37A) de l'écran inférieur (37) est adjacent à un bord inférieur (27A) de l'écran supérieur (27) lorsque l'orthèse de genou est dans la configuration droite.

15. Orthèse de genou (10) selon l'une quelconque des revendications précédentes, **caractérisée en ce que** la coupelle de protection (52) est pourvue d'une fente (57) à l'intérieur de laquelle l'élément de protection élastomère (54) est disposé de manière coulissante.

16. Orthèse de genou (10) selon la revendication 1, **caractérisée en ce que** la coupelle de protection (52) est disposée sur la surface interne de l'élément de protection élastomère (54).

17. Orthèse de genou (10) selon la revendication 1, **caractérisée en ce que** la coupelle de protection (52) est disposée sur la surface externe de l'élément de protection élastomère (54).
